# EUROPEAN PATENT SPECIFICATION

(11) **EP 2 195 030 B1**
(45) Date of publication and mention of the grant of the patent: **07.05.2014**
(21) Application number: 08806238.5
(22) Date of filing: 12.09.2008
(51) Int. Cl.: A61K 45/06, A61P 35/00, A61P 3/10, A61P 3/06, A61P 5/50, A61P 17/06, A61P 17/00, A61P 1/16, A61K 31/352

(54) **ISOFLAVONE FORMULATION**
ISOFLAVON-FORMULIERUNG
FORMULATION D'ISOFLAVONE

(30) Priority: 14.09.2007 US 960086 P
(43) Date of publication of application: 16.06.2010
(73) Proprietor: CamNutra Limited, Kings Norton Birmingham West Midlands B30 3JN (GB)
(72) Inventor: PETYAEV, Ivan, Cambridge Cambridgeshire CB1 7UY (GB); BASHMAKOV, Yuriy, Frisco, Texas 75035 (US)
(74) Representative: Sutcliffe, Nicholas Robert
(86) International application number: PCT/GB2008/003079
(87) International publication number: WO 2009/034325

(56) References cited:
- EP-A- 1 228 708
- WO-A-01/87315
- WO-A-02/067878
- WO-A-03/035086
- WO-A-03/049689
- US-A- 5 851 792
- US-A1- 2003 108 619
- US-A1- 2005 106 219
- DATABASE WPI Week 200501 Thomson Scientific, London, GB; AN 2005-008500 XP002510317 & KR 2004 071 349 A (LOTTE CONFECTIONERY CO LTD) 12 August 2004 (2004-08-12)
- ALLEN JERILYN K ET AL: "Effect of soy protein-containing isoflavones on lipoproteins in postmenopausal women." MENOPAUSE (NEW YORK, N.Y.) 2007 JAN-FEB, vol. 14, no. 1, January 2007 (2007-01), pages 106-114, XP009110472 ISSN: 1072-3714
- DAVIS JEREMY ET AL: "Soy protein and isoflavones influence adiposity and development of metabolic syndrome in the obese male ZDF rat." ANNALS OF NUTRITION & METABOLISM 2007, vol. 51, no. 1, 14 March 2007 (2007-03-14), pages 42-52, XP009110473 ISSN: 1421-9697
- DAVIS J ET AL: "Soy protein influences the development of the metabolic syndrome in male obese ZDFxSHHF rats" HORMONE AND METABOLIC RESEARCH, vol. 37, no. 5, May 2005 (2005-05), pages 316-325, XP009110484 ISSN: 0018-5043
- ARONSON WILLIAM J ET AL: "Decreased growth of human prostate LNCaP tumors in SCID mice fed a low-fat, soy protein diet with isoflavones" NUTRITION AND CANCER, vol. 35, no. 2, 1999, pages 130-136, XP009110482 ISSN: 0163-5581
- GUDBRANDSEN ODDRUN A ET AL: "Dietary proteins with high isoflavone content or low methionine-glycine and lysine-arginine ratios are hypocholesterolaemic and lower the plasma homocysteine level in male Zucker fa/fa rats" BRITISH JOURNAL OF NUTRITION, vol. 94, no. 3, September 2005 (2005-09), pages 321-330, XP009110477 ISSN: 0007-1145
- DATABASE WPI Week 200823, Derwent Publications Ltd., London, GB; AN 2008-D20784 & RU 2 309 603 C2 (ARCHER-DANIELS MIDLAND CO) 10 November 2007
- JING LEGANG ET AL: "Separation of protein and isoflavone in soybean whey by ultrafiltration.", YINGYONG SHENGTAI XUEBAO, vol. 17, no. 10, October 2006 (2006-10), pages 1993-1995, ISSN: 1001-9332
- AOYAMA T ET AL: "EFFECT OF SOY AND MILK WHEY PROTEIN ISOLATES AND THEIR HYDROLYSATES ON WEIGHT REDUCTION IN GENETICALLY OBESE MICE", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 64, no. 12, 1 December 2000 (2000-12-01), pages 2594-2600, XP001109501, ISSN: 0916-8451, DOI: DOI:10.1271/BBB.64.2594
- DATABASE WPI Week 200406, Derwent Publications Ltd., London, GB; AN 2004-056263, XP002317913 & JP 2003 299467 A (NICHIMO KK) 21 October 2003

## Description

This invention relates to the formulations of isoflavones which have improved biological activity and which may be useful in a range of therapeutic and medicinal applications.

Isoflavones are phytoestrogens which are found in plants such as soybeans (i.e. non-steroidal compounds that possess estrogen-like biological activity). Soy isoflavones have both weak estrogenic and weak anti-estrogenic effects. They have been found to bind to estrogen receptors-alpha (ER-alpha) and beta (ER-beta).

Metabolic abnormalities, such as insulin resistance, impaired glucose tolerance, hypertension, obesity and metabolic syndrome, have become increasingly common in the developed world and it is estimated that in America alone over 50 million people have such dysfunction. Metabolic dysfunctions are significant risk factors for the subsequent development of diabetes, cardiovascular disease, peripheral occlusive disease, cerebral and other forms of atherosclerosis.

WO 03/035086 A1, discloses soy proteins for treating type II diabetes.

DAVIS J ET AL, HORMONE AND METABOLIC RESEARCH, vol. 37, no. 5, May 2005, pp 316-325, discloses that soy protein improved metabolic parameters in obese rats.

US 2005/106219 A1, discloses a composition comprising mammalian whey proteins and soy isoflavones for increasing the bioavailability of a lipophilic bioactive agent.

Further development of effective treatments for these conditions would have a significant impact on the health of the world population.

The present inventors have found that isoflavones which are formulated with soluble carrier proteins have significantly increased ability to reduce levels of plasma lipids but do not display any increased estrogenic activity. The selective increase of non-estrogenic properties of isoflavones may be useful in modulating lipid metabolism, for example in the treatment of metabolic dysfunction, as well as in the enhancement of the anti-cancer and/or dermatological properties of isoflavones.

An aspect of the invention provides a composition comprising a soluble carrier protein i.e. mammalian whey protein and an isoflavone for use as defined in present claim 1.

A composition may comprise soluble carrier protein and an isoflavone in a weight ratio (carrier protein: isoflavone) of from 2:1 to 40:1, preferably 15:1 to 25:1, for example, about 20:1.

Suitable isoflavones may include isoflavone and isoflavone derivatives which possess similar biological properties.

Isoflavones are non-steroidal compounds derived from 3-phenyl-4*H*-1-benzopyr-4-one which occur naturally in plants, such as soybean and onions, and plant derived products such as tea and wine. Isoflavones have a range of different species and glycosylated forms. For example, an isoflavone for use as described herein may be an aglycone form such as genistein, daidzein and/or glycitein, or a glycosylated form, such as genistin, 6" -O-malonylgenistin and 6"-O-acetylgenistin, daidzin, 6"-O-malonyldaidzin, 6"-O-acetyldaidzin, glycitin, 6"-O-malonylglycitin and/or 6" -O-acetylglycitin

Isoflavones also include derivatives of natural isoflavones, such as dihydrodaidzein (DiD), *cis-* and *trans*-tetrahydrodaidzein and dehydroequol.

In some embodiments, a composition may comprise more than one isoflavone. For example, a composition may comprise multiple species or glycosylated forms of isoflavone.

An isoflavone for use as described herein may be natural i.e. obtained from a natural source, for example, extracted from a fungi or plant such as soybean, red clover, psoralea, kudzu, hops, lupine, fava, chick pea, alfalfa, or peanut. A range of methods for extracting, concentrating and/or purifying isoflavones from plants are known in the art. For example, isoflavones may be extracted by solvent extraction using ethanol, DMSO, ethyl acetate, hexane, acetone, soya or other vegetable oils, or non-vegetable oils.

An isoflavone for use as described herein may be synthetic i.e. produced by artificial means, for example, by chemical synthesis. A range of methods for chemical synthesis of isoflavones are known in the art (See for example: Hem Chandra Jha, et al. Angewandte Chemie International Edition 20. 1 102-103, 2003; Maruyama Masashi et al Nippon Kagakkai Koen Yokoshu, 81, 2, 1376, 2002).

Isoflavones are also available from commercial suppliers (e.g. Indena (Italy), Symrise (Germany), Holland & Barrett (USA/UK), Isovon (UK), Healthspan (UK), Biovea (UK)).

Derivatives of isoflavone as described above may be produced by chemical synthesis analogous to the synthesis described above or by chemical modification of natural isoflavone extracted from plant material.

Soluble carrier proteins include natural or synthetic hydrophilic peptides and polypeptides that are soluble in aqueous solution. Carrier proteins may be insoluble in organic solvents.

A composition described herein may comprise a single species of soluble carrier protein or a mixture of different soluble carrier proteins

Suitable soluble carrier proteins include dairy proteins. Suitable diary proteins may be conveniently obtained from milk, or other dairy fermented or fermented dairy products, such as yogurts, and cheeses.

The soluble carrier protein is mammalian whey protein. Conveniently, bovine whey protein may be employed.

Whey protein is a collection of globular proteins isolated from whey, which may be produced as a by-product of cheese manufacture. Whey protein is a mixture of beta-lactoglobulin (~65%), alpha-lactalbumin (∼25%), and serum albumin (∼8%), which are soluble in their native forms, independent of pH.

Suitable whey proteins may be obtained from a range of commercial sources.

A whey isolate suitable for use as described herein may comprise at least 60%, at least 70%, at least 80%, art least 90% or at least 95% whey protein.

A composition comprising carrier protein and isoflavone as described herein is produced by admixing the carrier protein with isoflavone under conditions sufficient to form a mixture. Carrier protein is dissolved in water and isoflavone is dissolved in an organic solvent, such as acetone, ethanol or isopropanol. The two solutions are then combined and the solvent evaporated to form a composition comprising carrier protein and isoflavone (termed 'lactoflavone' herein). A method of producing a composition as described herein comprises:
dissolving a soluble carrier protein such as whey protein in water,
dissolving one or more isoflavones in an organic solvent, such as acetone, ethanol or isopropanol,
mixing the two solutions, and
evaporating the solvent thereby producing the composition.

Conveniently, a solvent: water ratio of the order of 60:40 by volume may be employed when the aqueous phase is mixed with the solvent. A composition may comprise up to 50 % isoflavone and up to 90 % carrier protein.

Following evaporation, the composition may be in the form of a dispersion. The dispersion may be treated to produce the final form. For example, the dispersion may be heat-treated to produce a gel or dried, for example, by spraying or lyophilisation, to produce a powder.

The composition may then be formulated with carriers, excipients, stabilisers, sweeteners, additives, flavourings, colourings and/or emulsifiers as described above.
For example, a suitable lactoflavone formulation may comprise 2% (w/w) isoflavone, 20% (w/w) whey protein, 50.5%(w/w) microcrystalline cellulose, 5%(w/w) silicon dioxide, 3%(w/w) polysorbate 80 and 1.5%(w/w) soy lecithin.

Lactoflavone compositions may be administered in any convenient form or formulation.

Suitable formulations may display increased ability to reduce plasma lipid levels, increased ability to up-regulate LDL receptor expression, increased insulin sensitisation activity and/or increased ability to up-regulate insulin receptor levels.

Suitable formulations do not display increased estrogenic activity relative to isoflavone.

Lactoflavone compositions as described herein may conveniently be presented in unit dosage form and may be prepared by any methods well known in the art of pharmacy. Such methods include the step of bringing into association the active compound with the carrier which constitutes one or more accessory ingredients. In general, the formulations are prepared by uniformly and intimately bringing into association the active compound with liquid carriers or finely divided solid carriers or both, and then if necessary shaping the product.

Formulations may be in the form of liquids, solutions, suspensions, dispersions, emulsions, elixirs, syrups, tablets, lozenges, granules, powders, capsules, cachets, beads, pills, ampoules, suppositories, pessaries, ointments, gels, pastes, creams, sprays, mists, foams, lotions, oils, boluses, electuaries, or aerosols.

In some embodiments, a lactoflavone compound may be administered as a pharmaceutical composition comprising an isoflavone and a carrier protein as described above, together with one or more pharmaceutically acceptable carriers, adjuvants, excipients, diluents, fillers, buffers, stabilisers, emulsifiers, preservatives, lubricants, or other materials well known to those skilled in the art and, optionally, other therapeutic or prophylactic agents.

In some embodiments, a suitable pharmaceutical composition may comprise a lactoflavone as the sole active component. In other embodiments, a suitable pharmaceutical composition may comprise an isoflavone and one or more additional components, such as vitamins, carotenoids, sterols, phytosterols, retinoids, polyfenols, flavonoids, tocotrienols, terpenoids, statins, nuclear receptor ligands/agonists and other biologically active substances.

The term "pharmaceutically acceptable" as used herein pertains to compounds, materials, compositions, and/or dosage forms which are, within the scope of sound medical judgement, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation.

Suitable carriers, excipients, etc. can be found in standard pharmaceutical texts, for example, Remington's Pharmaceutical Sciences, 18th edition, Mack Publishing Company, Easton, Pa., 1990 and may include oils, for example vegetable oils, such as tomato oil, soya oil, or peanut oil, or non-vegetable oils, glycerol, gelatine, sucrose, glucose, ascorbyl palmitate, corn starch and silicon dioxide. Suitable emulsifiers include polysorbate 80. Suitable stabilisers may include sucrose ester, lecithin, antioxidants such as dl-a-tocopherol and ascorbyl palmitate, flavanoids, cellulose, waxes, mannitol, shellac, talc, calcium phosphate, magnesium stearate, and arabic or acacia gum. For example, isoflavones may contain about 10% isoflavones, 1.5% and 5.0%, respectively, of the antioxidants dl-a-tocopherol and ascorbyl palmitate, in addition to carrier substances such as vegetable oil, fish gelatine, sucrose and corn starch.

Formulations suitable for oral administration (e.g. by ingestion) may be presented as discrete units such as capsules, cachets or tablets, each containing a predetermined amount of the active compound; as a powder or granules; as a solution or suspension in an aqueous or non-aqueous liquid; or as an oil-in-water liquid emulsion or a water-in-oil liquid emulsion; as a bolus; as an electuary; or as a paste. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

A tablet may be made by conventional means, e.g., compression or moulding, optionally with one or more accessory ingredients. Compressed tablets may be prepared by compressing in a suitable machine the active compound in a free-flowing form such as a powder or granules, optionally mixed with one or more binders (e.g. povidone, gelatin, acacia, sorbitol, tragacanth, hydroxypropylmethyl cellulose); fillers or diluents (e.g. lactose, microcrystalline cellulose, calcium hydrogen phosphate); lubricants (e.g. magnesium stearate, talc, silica); disintegrants (e.g. sodium starch glycolate, cross-linked povidone, cross-linked sodium carboxymethyl cellulose); surface-active or dispersing or wetting agents (e.g. sodium lauryl sulfate); and preservatives (e.g. methyl p-hydroxybenzoate, propyl p-hydroxybenzoate, sorbic acid). Moulded tablets may be made by moulding in a suitable machine a mixture of the powdered compound moistened with an inert liquid diluent.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

Formulations suitable for topical administration (e.g. transdermal, intranasal, ocular, buccal, and sublingual) may be formulated as an ointment, cream, suspension, lotion, powder, solution, past, gel, spray, aerosol, or oil. Alternatively, a formulation may comprise a patch or a dressing such as a bandage or adhesive plaster impregnated with active compounds and optionally one or more excipients or diluents.

Formulations suitable for topical administration via the skin include ointments, creams, and emulsions. When formulated in an ointment, the active compound may optionally be employed with either a paraffinic or a water-miscible ointment base. Alternatively, the active compounds may be formulated in a cream with an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the active compound through the skin or other affected areas. Examples of such dermal penetration enhancers include dimethylsulfoxide and related analogues.

In other embodiments, a lactoflavone composition as described herein may be comprised in a food product. For example, it may be comprised in bread, cereal, biscuits, butter, sauces, bars, coatings, fillings, spreads (e.g. margarine), cheese, yogurts, chocolate, ice cream, nutritional supplements, enteral nutrition products or beverages. Other suitable food products will be apparent to a person skilled in the art.

The lactoflavone composition may be mixed with the ingredients of the food product prior to cooking (e.g. baking) and/or added to the food product after cooking.

In some embodiments, the isoflavone in the lactoflavone composition may not be naturally present in the food product.

A method of making a food product may comprise:
(i) providing a food product ingredient,
(ii) mixing a lactoflavone composition as described herein with said ingredient to produce a mixture and
(iii) formulating said mixture into a food product.

In other embodiments, a lactoflavone composition as described herein may be comprised in a cosmetic product. For example, it may be comprised in a cream, lotion, paste or other ointment which is topically applied to the skin. Other suitable cosmetic products will be apparent to a person skilled in the art.

A method of making a cosmetic product may comprise:
(i) providing an ingredient of a cosmetic product,
(ii) mixing a lactoflavone composition as described herein with said ingredient to produce a mixture and
(iii) formulating said mixture into a cosmetic product.

Lactoflavone compositions and formulations as described herein may be useful in the treatment of metabolic dysfunction.

Metabolic dysfunction may include any disease or condition associated with abnormal lipid or glucose metabolism. Metabolic dysfunction may include obesity, hyperlipidemia, insulin resistance, reduced glucose tolerance, polycystic ovary syndrome (PCOS), hypertension, liver disorders, such as steatosis, chronic hepatitis, liver fibrosis and cirrhosis, and metabolic syndrome.

Obesity is a condition characterised by excess body fat. For example, an obese individual may have a body mass index (BMI: Mass/Height²) of greater than 30. Obesity is a risk factor for a range of medical conditions, including diabetes and cardiovascular disorders such as atherosclerosis, ischaemic (coronary) heart disease, myocardial ischaemia (angina) and stroke. Obesity may include abdominal obesity, which is commonly defined as waist circumference more than 94 cm for European men and more than 80 cm for European women, more than 90 cm for South Asian men and more than 80 cm for South Asian women, and more than 85 cm for Japanese men and more than 90 cm for Japanese women. The methods described herein may be useful in weight control and the treatment of obesity.

Insulin resistance is commonly defined as being within the highest quartile for the relevant population and is present in 25% of the non-diabetic population, with an estimated conversion rate from an insulin resistant state to type 2 diabetes of 2-12% per year.

Reduced glucose tolerance is commonly defined as a glucose concentration > 7.8 mmol-1 at 120 min of the oral glucose tolerance test. Reduced glucose tolerance is associated with insulin resistance and hypoglycaemia.

Polycystic ovary syndrome (PCOS) is an endocrine disorder that affects 5-10% of women, in which cysts in the ovary interfere with normal ovulation and menstruation. PCOS is associated with insulin resistance, compensatory hyperinsulinaemia, metabolic syndrome, obesity, and hyperandrogenaemia.

Hypertension is characterised by a consistently elevated blood pressure which exceeds 140 over 90 mmHg (>140 systolic pressure; > 90 diastolic pressure).

Liver disorders include steatosis, chronic hepatitis, liver fibrosis and cirrhosis. Liver steatosis is characterised by an abnormal lipid increase in the liver which is caused by reduced oxidation of fatty acids or decreased synthesis and release of lipoproteins. Steatosis may subsequently transform into liver fibrosis and cirrhosis. Liver fibrosis is characterised by the growth of fibrous tissue in the liver. Fibrosis can lead to cirrhosis, in which the liver has reduced function and becomes permanently scarred, fibrous, and fatty.

Chronic hepatitis is an inflammation of the liver which may be caused by bacterial or viral infection, parasitic infestation, alcohol, drugs, toxins, or transfusion of incompatible blood

Metabolic syndrome is a condition which is characterised by obesity and one or more of; insulin resistance, high blood pressure, high blood triglyceride levels and/or low HDL cholesterol levels in the blood. For example, the IDF (International Diabetes Federation) defines metabolic syndrome as: abdominal obesity plus any two of the following: raised triglyceride levels (above 1.7 mmol/L); reduced HDL cholesterol (below or at 0.9 mmol/L in men and below or at 1.1 mmol/L in women); raised blood pressure (above 130/85) and raised fasting plasma glucose (above 5.6 mmol/L).

Metabolic dysfunction may also include hypercholesterolaemia, low total and/or LDL cholesterol (for example, <0.9mmoll⁻¹, men; <1.0mmoll⁻¹, women), hypertriglyceridaemia (for example, plasma TAG's ≥1.7mmoll⁻¹) and microalbuminuria (for example, urinary albumin excretion rate ≥ 20 µg min⁻¹; albumin: creatinine ratio ≥ 30 mg min⁻¹).

In some embodiments, metabolic dysfunction may include diabetes (e.g. type 1 or type 2 diabetes).

In other embodiments, an individual treated in accordance with the present methods is not diabetic (i.e. is not suffering from type 1 or type 2 diabetes) and/or is not suffering from an atherosclerotic condition, such as CHD, stroke or peripheral vascular disease.

In some embodiments, metabolic dysfunction may include an atherosclerotic condition, such as CHD, stroke or peripheral vascular disease coronary heart disease (CHD) and other atherosclerotic conditions. In some embodiments, an individual with a metabolic dysfunction may have an increased risk factor of suffering from such a condition.

The methods described herein may be useful in the treatment of metabolic dysfunction. For example, a method of treating metabolic dysfunction may comprise;
administering a lactoflavone composition as described herein to an individual in need thereof.

A suitable lactoflavone composition may be produced by a method described above.

Metabolic dysfunction is described in more detail above.

Methods described herein may also have prophylactic applications. For example, a method of preventing or delaying the onset of metabolic dysfunction may comprise administering a lactoflavone composition as described herein to an individual in need thereof.

An individual suitable for undergoing a method of preventing or delaying the onset of a metabolic dysfunction as described herein may be at risk of or susceptible to metabolic dysfunction, for example the individual may have one or more risk factors associated with the onset of metabolic dysfunction.

Another aspect of the invention provides the use of a lactoflavone composition as described herein in the manufacture of a medicament for use in the treatment of metabolic dysfunction as described above, or preventing or delaying the onset of metabolic dysfunction.

Another aspect of the invention provides a lactoflavone composition as described herein for use in the treatment of metabolic dysfunction and a lactoflavone composition as described herein for use in preventing or delaying the onset of metabolic dysfunction.

Lactoflavone compositions and formulations as described herein may be useful in the treatment of cancer (not claimed).

The methods described herein may be useful in the treatment of cancer. For example, a method of treating cancer may comprise;
administering a lactoflavone composition as described herein to an individual in need thereof.

A suitable lactoflavone composition may be produced by a method described above.

Methods described herein may also have prophylactic applications. For example, a method of preventing or delaying the onset of cancer may comprise administering a lactoflavone composition as described herein to an individual in need thereof.

An individual suitable for undergoing a method of preventing or delaying the onset of cancer as described herein may be at risk of or susceptible to cancer, for example the individual may have one or more risk factors associated with the onset of cancer.

Cancers may include any type of solid cancer for example, sarcomas, skin cancer, bladder cancer, breast cancer, uterine cancer, ovarian cancer, prostate cancer, lung cancer, colorectal cancer, cervical cancer, liver cancer, head and neck cancer, oesophageal cancer, pancreatic cancer, renal cancer, stomach cancer and cerebral cancer or lymphoid cancers, such as leukaemia or lymphoma.

Another aspect of the disclosure provides the use of a lactoflavone composition as described herein in the manufacture of a medicament for use in the treatment of cancer as described above, or preventing or delaying the onset of cancer.

Another aspect of the disclosure provides a lactoflavone composition as described herein for use in the treatment of cancer and a lactoflavone composition as described herein for use in preventing or delaying the onset of cancer.

Lactoflavone compositions and formulations as described herein may be useful in the modulation of the properties of skin and the treatment of skin conditions and dysfunction.

The methods described herein may be useful in the modulation of the properties of skin. For example, a method of modulating the properties of skin may comprise;
administering a lactoflavone composition as described herein to an individual in need thereof.

Skin properties may be modulated for therapeutic purposes (e.g. the treatment of skin conditions and dysfunction) or non-therapeutic (e.g. cosmetic) purposes.

Methods described herein may also have prophylactic applications. For example, a method of preventing or delaying the onset of a skin condition or dysfunction may comprise administering a lactoflavone composition as described herein to an individual in need thereof.

An individual suitable for undergoing a method of preventing or delaying the onset of a skin condition or dysfunction as described herein may be at risk of or susceptible to a skin condition or dysfunction for example the individual may have one or more risk factors associated with the onset of a skin condition or dysfunction.

Skin conditions or dysfunction may include inflammatory skin conditions, such as eczema and psoriasis.

Another aspect of the disclosure provides the use of a lactoflavone composition as described herein in the manufacture of a preparation for use in the treatment of a skin condition or dysfunction as described above, or preventing or delaying the onset of a skin condition or dysfunction.

Another aspect of the disclosure provides a lactoflavone composition as described herein for use in modulating skin properties, for example in the treatment of a skin condition or dysfunction and a lactoflavone composition as described herein for use in preventing or delaying the onset of a skin condition or dysfunction.

Administration of a lactoflavone composition is preferably in a "therapeutically effective amount", this being sufficient to show benefit to the individual. It will be appreciated that appropriate dosages of the active compounds, and compositions comprising the active compounds, can vary from patient to patient. Determining the optimal dosage will generally involve the balancing of the level of therapeutic benefit against any risk or deleterious side effects of the treatments of the present invention. The selected dosage level will depend on a variety of factors including, but not limited to, the activity of the particular compound, the route of administration, the time of administration, the rate of excretion of the compound, the duration of the treatment, other drugs, compounds, and/or materials used in combination, and the age, sex, weight, condition, general health, and prior medical history of the patient. The amount of compound and route of administration will ultimately be at the discretion of the physician, although generally the dosage will be to achieve local concentrations at the site of action which achieve the desired effect without causing substantial harmful or deleterious side-effects.

Administration in vivo can be effected in one dose, continuously or intermittently (e.g. in divided doses at appropriate intervals) throughout the course of treatment. Methods of determining the most effective means and dosage of administration are well known to those of skill in the art and will vary with the formulation used for therapy, the purpose of the therapy, the target cell being treated, and the subject being treated. Single or multiple administrations can be carried out with the dose level and pattern being selected by the treating physician.

In general, a suitable dose of lactoflavone is in the range of about 100 µg to about 250 mg per kilogram body weight of the subject per day. Where the active compound is a salt, an ester, pro-drug, or the like, the amount administered is calculated on the basis of the parent compound and so the actual weight to be used is increased proportionately.

A lactoflavone composition as described herein may be administered alone or in combination with other treatments, either simultaneously or sequentially dependent upon the condition to be treated.

For example, a method described herein may comprise administering a lactoflavone composition as described herein in combination with a statin (i.e. a 3-Hydroxy-3-methylglutaryl Coenzyme A (HMG Co A) reductase inhibitor).

Suitable statins include pravastatin (PRAVACHOL^{™}), lovastatin (MEVACOR^{™}), simvastatin (ZOCOR^{™}), cerivastatin (LIPOBAY^{™}), fluvastatin (LESCOL^{™}), atorvastatin (LIPITOR^{™}), mevastatin and rosuvastatin (Crestor^{™}). Other suitable statins are known in the art and may be readily identified by using HMG-CoA reductase assays which are well known in the art. Examples of such assays are disclosed in U.S. Pat. No.4,231,938.

A composition or medicament comprising a lactoflavone composition as described herein may further comprise a statin (i.e. a 3-Hydroxy-3-methylglutaryl Coenzyme A (HMG Co A) reductase inhibitor.

Examples of suitable statins are described above. Other statins useful in the methods of the present invention will be apparent to the skilled person.

Another aspect of the invention is a kit comprising a lactoflavone composition as described herein and a statin for use in combination to treat metabolic dysfunction.

A kit may further include one or more other articles and/or reagents for performance of the method described herein, such as means for administering the lactoflavone composition as described herein and/or statin, e.g. a syringe (such components generally being sterile).

The kit may further include instructions for carrying out all or part of the method of the invention, e.g. the dosage regime for the lactoflavone and, optionally, the statin.

Yet another aspect of the invention is use of a combination of a lactoflavone composition as described herein and a statin in the manufacture of a medicament for the treatment of metabolic dysfunction as described above, or preventing or delaying the onset of a metabolic dysfunction.

Various further aspects and embodiments of the present invention will be apparent to those skilled in the art in view of the present disclosure.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

Certain aspects and embodiments of the invention will now be illustrated by way of example and with reference to the figures described above and tables described below.
Figure 1 shows the induction of AKT phosphorylation in HUVEC cells after treatment with soy isoflavones.
Figure 2 shows total AKT levels in HUVEC cells after treatment with soy isoflavones.
Figure 3 shows plasma triglyceride levels in mice fed on a high fat diet. Group 1 - control, group 2 - untreated, group 3 soy isoflavone treated, group 4 lactoflavone treated.
Figure 4 shows plasma cholesterol levels in mice fed on a high fat diet. Group 1 - control, group 2 - untreated, group 3 soy isoflavone treated, group 4 lactoflavone treated.
Table 1 shows folds of mRNA changes in rat primary hepatocytes treated with whey protein, soy isoflavones and preferred composition containing soy isoflavones and whey protein
Table 2 shows folds of mRNA changes in mouse livers treated with high-fat diets.

### Experiments

### Materials

HUVEC cultured cell line (CRL-1730) was obtained from ATCC (USA). Soy isoflavones were purchased from Holland & Barrett Ltd (USA) as a standardized concentrate containing 3% isoflavones. Molar concentrations of soy isoflavones were referred to molecular weight of genisitin. Antibody against AKT, phospho-AKT (Ser 473) and immunoprecipitation reagents were from Cell Signalling Technology, (USA).

### Production of lactoflavones

Lactoflavone was produced by preparing a solution of whey protein in water and a solution of soy isoflavone in acetone. The two solutions were mixed and stirred and the solvents evaporated to produce the lactoflavone formulation.

In the particular case, 20g of soy isoflavones were stirred overnight at 30°C in 650 ml of acetone. At the same time, 200g of whey protein was dissolved in 650 ml of deionized water. The solutions were combined under continuous stirring by slow addition of soy isoflavones solution to whey protein solution. The obtained mixture was stirred overnight at 4°C. The final composition was dried under vacuum until remains of water are driven off. The resulting lactoflavone powder was used in the experiments described below.

### Cultured cells

HUVECs (CRL-1730, ATCC, USA)) were grown in Ham's F12K medium with 2 mM glutamine supplemented with 10 % fetal bovine serum and 0.1 mg/ml heparin. The cells were incubated at 37 C in presence of 5% CO2 until confluency reached 70-80 %. 24 hours prior insulin addition the cells were switched to serum free medium. Additions of insulin (100 nM), soy isoflavones (50 µM) or primary composition which includes soy isoflavones and whey protein (50 µM of isoflavones) were made to serum free medium 5 min prior harvesting the cells. All treatment groups contained trace amounts of DMSO (10 µL per 10 ml of medium), which was used as a solvent for soy isoflavones.

### Phospho-AKT response

To evaluate the intensity of AKT phosphorylation response in different treatment groups, site-specific antibody, directed to phosphorylated serine residue (Ser 473) in AKT molecule was used (Phospho-AKT, Ser 473, Cell Signaling Technology, USA). 5 minutes after after additions, the cells were washed with ice-cold PBS supplemented with 1 mM sodium ortovanadate and harvested in the cell lysis buffer (20 mM Tris-HCl (pH 7.5), 150 mM NaCl, 1% Triton X-100, 2 mM Sodium pyrophosphate, 1 mM β-glycerophosphate, 1 mM sodium ortovanadate, 1 µg/ml leupeptin. Cells were sonicated wih a seringe on ice. Total cell lysate (50 µg of protein) were run in 8% reduced SDS-PAGE. Membranes were incubated with phospho-AKT (Serine 473) specific antibody. Loading control originated from the stripped membranes incubated with antibody against total AKT (#9272, Cell Signaling Technology, USA). Filters were incubated with HRP-conjugated secondary antibody, washed and exposures were taken with X-ray films after incubation with LumiGLO (Cell Signaling Technologies, USA) substrate.

### Immunoprecipitation protocol

Immunoprecipitation study has been performed to confirm immunoblot data obtained from total lysates of HUVEC cells treated with insulin or different formulations of soy isoflavones. In both cases conditions for cell cultivation and treatment were the same. Cells were harvested at the same time and temperature in the cell lysis buffer mentioned above. After sonication, and 5 min centrifugation at 15.000 rpm/min, 200 µl of cleared cell lysate was mixed and incubated overnight with phospho-AKT (Serine 473) primary polyclonal antibody (1:100 of commercial solution, #9271, Cell Signaling Technologies, USA). Incubation was performed at 4 C on rotating wheel. Immune complexes were immobilized on protein A agarose beads, which were added to cell lysates on the amount of 20 µL of 50% bead slurry and incubated on rotating wheel for 2 hours at 4 C. After incubation beads were washed three times with 500 µl ice-cold cell lysis buffer and bound protein was eluted from the beads with 20 µl 3x SDS sample buffer. Heat-denatured protein samples were run in 10% SDS-PAGE. HUVEC cell lysates incubated with irrelevant primary antibody (antibody against CREBP were used as negative control for the immunoprecipitation experiment). The membranes were blotted overnight with monoclonal antibody against phospho-AKT (Ser 473, #4051, Cell Signaling Technologies, USA) and were developed as mentioned above.

### Primary Hepatocyte Study

Primary hepatocytes were isolated from rat liver of non-fasted Spraque-Dawley rats by collagenase digestion method. Under halothane anesthesia each liver was perfused in situ through portal vein subsequently with Liver perfusion medium and Liver Digest Medium (Gibco/BRL, USA). Livers were excised, liver tissue dissociated and hepatocytes plated onto 100 mm collagen I -coated dishes (Becton Dickinson Labware, USA) at density 6 million cells per dish in DMEM containing 5% Fetal Calf Serum with penicillin (100 units/ml) and streptomycin (100 µg/ml). After 3 hours attachment the cells were switched to serum-free DMEM, supplemented with 100 nM dexamethasone, 1 nM insulin, 100 nM triiodthyronine, 100 units/ml penicillin, 100 µg/ml streptomycin. Viable cells were counted trypan blue exclusion test. Hepatocytes with at least 85% of viable cells were used for studies. After overnight incubation, addition of different formulations of soy isoflavones at final concentration of 50 µM were made to the medium. All dishes received addition of DMSO (10 µl/10 ml of medium). RNA isolation has been done 18 hours after supplementation of hepatocytes with soy isoflavones.

### Results

### Lactoflavone induces Phospho-Akt Phosphorylation at Serine 473 In Cul tured HUVECS

Soy polyphenolic isoflavones are structural analogs of mammalian estrogens and capable of binding to estrogen receptors (ERα and ERβ). Their interaction with estrogen receptors is known to lead to the activation of downstream signaling pathway which includes estrogen receptor kinases, PI-3- kinase and AKT. In order to validate receptor binding and activation of the downstream signalling pathway, immortalized human endotheliocytes were incubated with the lactoflavone composition comprising soy isoflavones and whey protein. The phospho-AKT response at the serine residue 473 upon addition of soy isoflavones was compared to the insulin-induced AKT phosphorylation, which is a classic and well understood signalling mechanism in the insulin sensitive cell lines.

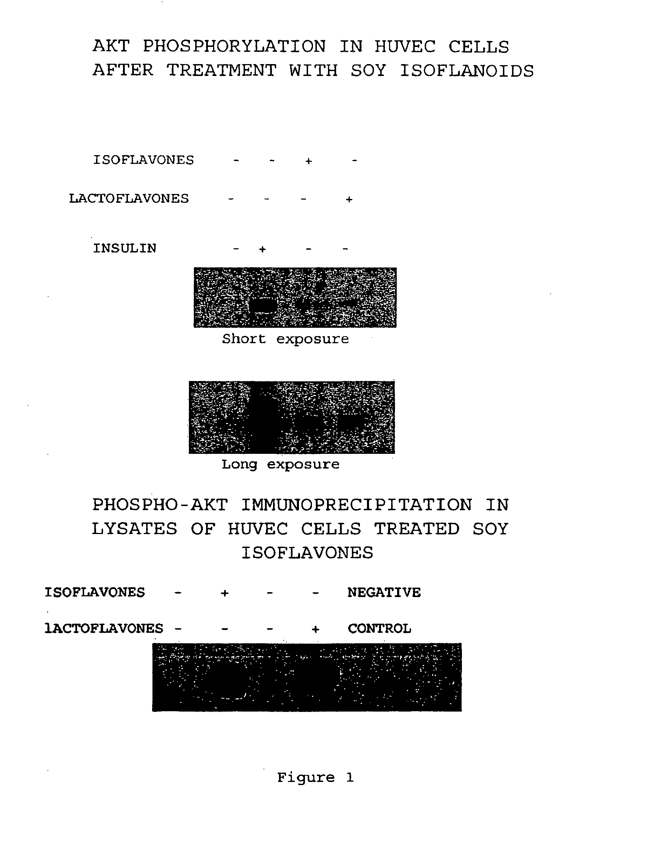

As can be seen from Fig. 1, treatment of HUVEC cells with 100 nM insulin lead to a significant changes in the amount of phosphorylated form of the enzyme. Notably, basal level of phospho-AKT in untreated HUVEC cells is very low and hardly detectable. Both forms of isoflavones - with (last lane) or without (3rd lane) whey protein had a similar effect on phospho-AKT induction when used at same concentration (50 µM). Soy isoflavones were found to be less potent, but still considerable inducers of AKT phosphorylation relative to insulin. Immunoprecipitation results, represented at the lower part of the figure 1 confirm that conclusion. The same amount of immunoprecipitated phospho-AKT was observed in the cells treated with pure isoflavones and the cells treated with lactoflavones. When irrelevant (CREP specific) antibody was used for immunoprecipitation, no phospho-AKT was detected in the IP pellets (negative control). The formulation of isoflavone into a lactoflavone composition as described herein therefore has no effect on the interaction of isoflavone with estrogen receptors and the subsequent activation of the downstream estrogen signalling pathway which includes estrogen receptor kinases, PI-3- kinase and AKT.

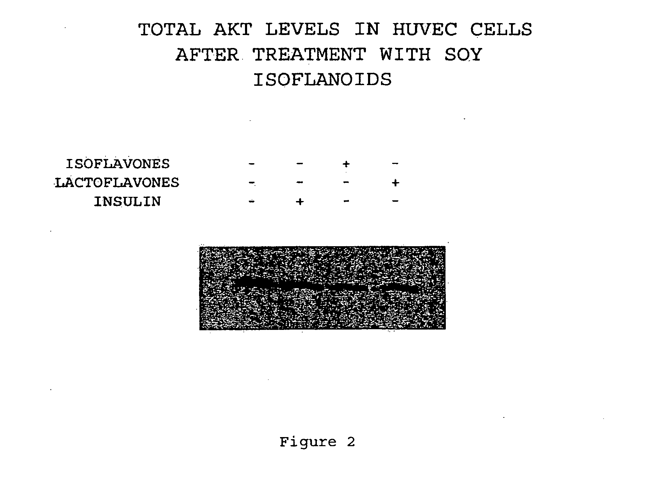

Figure 2 shows that total AKT level is unaffected in all experiments groups, and might serve as loading control for the study.

### Lactoflavone Upregulates LDL-Receptor mRNA in Primary Rat Hepatocytes

In order to evaluate the effect of the lactoflavone composition on mRNA levels in rat primary hepatocytes, the cells were treated with its two major ingredients - whey protein (table 1, second column) and soy isoflavones (table 1, third column). These changes were referred to the CT values of the genes in the cells kept in the serum free conditions. All mRNA values were normalized to the CT values of major housekeeping gene- 36B4. As can be seen from the table 1, 36B4 CT numbers in all experimental groups varied from 25.06 to 25.99, which indicates the overall health of the cell monolayers and absence of drug toxicity.

In general, addition of whey protein to the medium changed most of the CT values of the genes of interest. Changes in the colloidal oncotic pressure of the medium are known to induce multiple transcriptional abnormalities in the cultured cells including activation of the SREBP pathway.

As can be seen from table 1, the basal level of two major lipogenic mRNAs SREBP-1c and SREBP-2, was well above control value in protein-supplemented medium as compared to the hepatocytes kept in the serum-free conditions. These values reached highest levels in the cells treated with the lactoflavone composition. mRNA for a major gene of interest - LDL-receptor followed the same pattern. Its maximum activation, exceeding control over 3 fold, was observed when the cells were treated with the lactoflavone composition. Similar activation has been seen for mRNAs of FAS and ACS, two other downstream lipogenic genes belonging to SREBP pathway. However, at the time point used, we could not see significant increase in mRNAs for HMG-CoA reductase and INSIG-1.

Another important observation is related to the mRNA values of insulin-related genes. As can be seen in the table 1, soy isoflavones activate insulin receptor mRNA, with greatest activation shown by the lactoflavone composition. Despite the relative modesty of insulin receptor mRNA induction, two major insulin sensitive genes - PEPCK and IGFBP were repressed at most, when the lactoflavone composition was added to the cells. This pattern of mRNA changes is attributable to an increased insulin sensitivity of rat primary hepatocytes treated with the lactoflavone composition. The results set out herein show that the lactoflavone composition has an enhanced ability to up-regulate LDL receptor mRNA and improves insulin sensitivity in the primary hepatocytes. An increased degree of favourable metabolic changes in the hepatocytes treated with lactoflavones was hardly predictable and unexpected since pure isoflavones and lactoflavones were found to have similar ability to activate phosphorylation of AKT in the HUVECs.

### Lactoflavone Composition Reduces Plasma Lipids And Upregulates Hepatic LDL-Receptor In Mice Treated With High-Fat Diet

Animal study presented below reveals the functional significance of LDL-receptor up-regulation seen in primary hepatocytes treated with preferred primary composition of soy isoflavones.

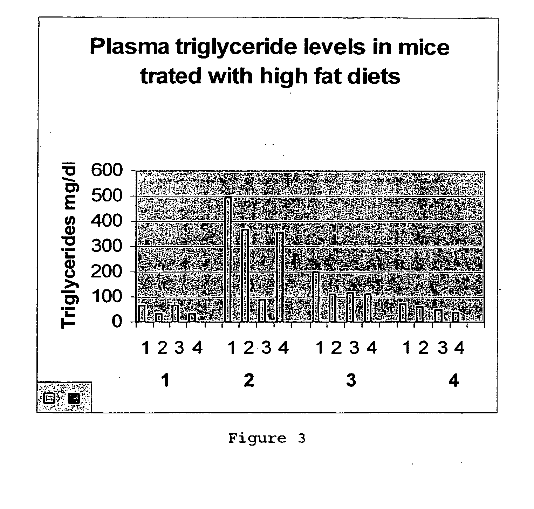

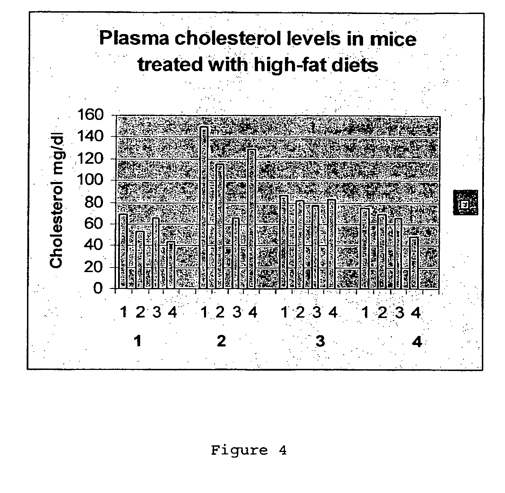

Figures 3 and 4 show that a four week high-fat diet regime leads to significant induction of plasma cholesterol and triglyceride levels in mice. This increase was more profound for plasma triglyceride level. Most of the mice (second group, Figure 3) developed hypertriglyceridemia exceeding 300 mg/dl, whereas plasma cholesterol level was induced in those animals just about two folds (Figure 4).

Supplementation of the high-fat diet with soy isoflavones ameliorated induction of both plasma triglyceride and plasma cholesterol levels (third group in Figures 3 and 4). The average triglyceride value in mice kept on high -fat diet with isoflavones reached 135.8 mg/dl, whereas that level was significantly higher in the untreated mice on high fat diet (average - 327.0 mg/dl).

However, the lactoflavone composition was found to have a much more powerful effect than pure isoflavone (Figure 3). Addition of the lactoflavone composition reduced the average value for plasma triglycerides to 55.4 mg/dl, versus 135.8 mg/dl in mice treated with the standard (pure) formulation of isoflavones. The average value for plasma cholesterol level in these two groups dropped less significantly, but still noticeably (from 81.8 mg/dl in pure isoflavone group, to 64.6 mg/dl in lactoflavone group, Figure 4).

The lactoflavone composition described herein was found to display increased potency in the reduction of plasma lipids in the mice treated with high-fat diet than a pure isoflavone formulation.

To understand the mechanism lipid-lowering effect of the lactoflavone formulation, we have analyzed changes of major hepatic mRNAs in mice treated with high-fat diet supplemented with different isoflavone formulations.

As can be seen from Table 2, none of treatment regimens affected absolute CT values for mayor housekeeping gene - 36B4 in the mouse liver. This demonstrates the absence of toxicity and the relatively healthy status of the animals used in the study.

However there are significant changes in mRNA levels of mayor lipidogenic genes. SREBP-1c mRNA was upregulated - 4 folds in the livers of mice kept on high fat diet. This is explained by significant amount of cholesterol ingested with a diet. It is well known, that dietary cholesterol activates the hepatic LXR pathway, leading to transcriptional activation of LXR-target genes. As a consequence, the high-fat diet induces some other LXR-target genes, including SCD-1 mRNA (- 2 fold over control value) and ABCg5 mRNA (-1.6 fold over control). Other lipogenic genes, including FAS, ME and ACS were slightly induced in the livers of mice on high fat diet, which is due to the direct affect of SREBP-1c on hepatic lipogenesis. However, the high-fat diet repressed significantly mRNA transcripts for mayor cholesterol biosynthetic enzymes - HMG-CoA reductase (44% reduction) and especially HMG-CoA synthase (82% reduction). High-fat diet enriched with cholesterol, may directly affect endogenous cholesterol biosynthetic pathway and transcription of cholesterologenic enzymes by overaccumulation of cholesterol in the hepatocytes. This is indicated by the drop in the hepatic LDL-receptor mRNA which was observed in the mice kept on high-fat diet. It is well documented elsewhere that high cholesterol intake and accumulation of cholesterol in the hepatic cells may lead to the reduction of LDL-receptor transcription. Decline in the hepatic LDL-receptor mRNA in cholesterol-fed animals is widely considered as a direct effect of cholesterol on LDL-R mRNA turnover, as well as indirect influence of cholesterol on SREBP-2 transcription. SREBP-2 is a mayor transcriptional factor controlling endogenous pathway of cholesterol biosynthesis and LDL-receptor in the animal liver. It has to be noted, that high-fat feeding significantly reduced SREBP-2 mRNA ∼ 74%. Thus, the changes in cholesterol metabolism in the livers of high-fat diet fed mice can be considered as a secondary phenomenon, predetermined by reduced level of SREBP-2.

As can be seen from Table 2, treatment with high-fat diet also leads to the reduction of insulin receptor mRNA (- 30 % of control level) and corresponding induction of PEPCK mRNA in the mouse liver.

Most significant observation comes from last two groups of the studies, when animals were kept on high-fat diet supplemented with different formulations of soy isoflavones. First and major observation is related to LDL-receptor mRNA. Both isoflavone formulations, especially lactoflavones enhanced LDL-receptor mRNA levels, which is consistent with the fact of plasma lipid normalization seen in the experimental animals.

Secondly, introduction of soy isoflavones reduced significantly some of the genes involved in triglyceride biosynthesis (SREBP-1c, FAS, SCD-1) in mice kept on high-fat diet. However, that anti-lipogenic effect was partial since some other lipogenic mRNA (malic enzyme, ACS) remained slightly induced. This may explain the incomplete effect on plasma triglyceride level reduction in mice treated with soy isoflavones. Higher isoflavone concentration and/or longer treatment might be required to see more profound isoflavone effect on plasma lipids. Despite the partial lipid-lowering effect, the lactoflavone composition showed the most favorable effect on genes involved in hepatic lipogenesis.

Thirdly, supplementation of high-fat diet with the lactoflavone composition leads to the normalization of insulin receptor levels in the livers of the experimental mice. As a result, 2 major insulin-, sensitive genes - IRS-2 and PEPCK were significantly down-regulated (reduction - 60%).

Thus supplementation of high-fat diet with the lactoflavone composition leads to a lipid-lowering effect due to:
1. Normalization of hepatic LDL-receptor transcription
2. Repression of transcriptional regulation of hepatic lipogenic system.

The lactoflavone formulation described herein has been shown to possess a unique ability to up-regulate insulin receptor transcription, which leads to an increased insulin sensitivity, without any increased estrogenic activity. The data set out herein provides theoretical and experimental basis for anti-diabetogenic properties of lactoflavone compositions, as well as their use in management of metabolic dysfunction, including conditions such as metabolic syndrome and diabetes mellitus.

**Table 1**

| Gene | Serum free medium | Whey protein | Isoflavones | Soy Isoflavones+ whey protein |
|---|---|---|---|---|
| 36B4ct | 25.06 | 25.73 | 25.99 | 25.68 |
| | | | | |
| LDL-R | 1 | 1.69 | 2.57 | 3.33 |
| SREBP-1c | 1 | 4.7 | 7.24 | 9.32 |
| SREBP-2 | 1 | 1.41 | 1.67 | 2.14 |
| HMG-Red | 1 | 1.1 | 1.2 | 1.15 |
| INSIG-1 | 1 | 0.32 | 1.08 | 1.27 |
| FAS | 1 | 4.7 | 6.89 | 9.47 |
| ACS | 1 | 2.76 | 3.77 | 5.1 |
| IR | 1 | 3.23 | 4.45 | 8.42 |
| PEPCK | 1 | 1.63 | 0.85 | 0.65 |
| IGFBP | 1 | 1 | 0.48 | 0.36 |

**Table 2**

| Gene | ct | Control | High-Fat | High-Fat Isoflavones | High-Fat Lactoflavones |
|---|---|---|---|---|---|
| 36B4 ct | | 20.93 | 20.95 | 20.97 | 21.02 |
| | | | | | |
| SREBP-1c | 21.68 | 1 | 4.28 | 1.07 | 0.67 |
| SREBP-2 | 21.68 | 1 | 0.26 | 0.43 | 0.57 |
| | | | | | |
| SCD-1 | 20.1 | 1 | 2.18 | 0.66 | 0.65 |
| FAS | 19.8 | 1 | 1.52 | 0.57 | 0.18 |
| INSIG-1 | 23.9 | 1 | 1.58 | 0.1 | 0.64 |
| ME | 24.06 | 1 | 1.37 | 1.95 | 1.91 |
| ACS | 26.8 | 1 | 1.46 | 2.53 | 1.7 |
| HMG Syn | 22.01 | 1 | 0.18 | 0.4 | 0.76 |
| HMG Red | 22.18 | 1 | 0.56 | 0.23 | 0.32 |
| LDL-R | 22.17 | 1 | 0.64 | 1.47 | 1.7 |
| ABCg5 | 23.68 | 1 | 1.63 | 0.75 | 0.95 |
| ABCg8 | 24.59 | 1 | 0.73 | 1.21 | 0.88 |
| IRS-2 | 25.18 | 1 | 0.75 | 0.73 | 0.34 |
| PEPCK | 19.18 | 1 | 1.82 | 0.96 | 0.41 |
| IR | 21.92 | 1 | 0.67 | 0.34 | 1.23 |

## Claims

1. A composition comprising mammalian whey protein and an isoflavone for use in a method of treatment of metabolic dysfunction associated with abnormal lipid or glucose metabolism,
wherein the composition is producible by a method comprising;
dissolving mammalian whey protein in water,
dissolving one or more isoflavones in an organic solvent,
mixing the two solutions, and
evaporating the solvents to produce the composition,
wherein the metabolic dysfunction is associated with abnormal lipid or glucose metabolism.

2. A composition for use according to claim 1 wherein the isoflavone is selected from the group consisting of genistein, daidzein, glycitein.

3. A composition for use according to claim 1 or claim 2 wherein the isoflavone is glycosylated.

4. A composition for use according to claim 3 wherein the glycosylated isoflavone is selected from the group consisting of genistin, 6" -O-malonylgenistin, 6"-O-acetylgenistin, daidzin, 6"-O-malonyldaidzin and 6"-O-acetyldaidzin, glycitin, 6"-O-malonylglycitin and 6" -O-acetylglycitin.

5. A composition for use according to any one of the preceding claims which comprises more than one isoflavone.

6. A composition for use according to any one of the preceding claims which displays one or more of increased ability to reduce plasma lipid levels, increased ability to upregulate LDL receptor expression, increased ability to up-regulate insulin receptor expression and/or increased insulin sensitisation activity relative to isoflavone.

7. A composition for use according to any one of the preceding claims which does not display increased estrogenic activity relative to the isoflavone.

8. Use of a composition comprising mammalian whey protein and an isoflavone in the manufacture of a preparation for use in treating metabolic dysfunction associated with abnormal lipid or glucose metabolism., wherein the composition is producible by a method comprising;
dissolving mammalian whey protein in water,
dissolving one or more isoflavones in an organic solvent,
mixing the two solutions, and
evaporating the solvents to produce the composition.

9. A composition for use according to any one of claims 1 to 7 or a use according to claim 8 wherein the metabolic dysfunction is selected from the group consisting of diabetes, obesity, insulin resistance, reduced glucose tolerance, polycystic ovary syndrome, hypertension, steatosis, chronic hepatitis, liver fibrosis, cirrhosis, hypercholesterolaemia, hypertriglyceridaemia, microalbuminuria and metabolic syndrome.

## Patentansprüche

1. Zusammensetzung, die Säugetier-Molkenprotein und ein Isoflavon umfasst, zur Verwendung in einem Verfahren zur Behandlung von Stoffwechselfehlfunktion im Zusammenhang mit anomalem Lipid- oder Glucosestoffwechsel,
worin die Zusammensetzung durch ein Verfahren herstellbar ist, das Folgendes umfasst:
das Lösen von Säugetier-Molkenprotein in Wasser,
das Lösen eines oder mehrerer Isoflavone in einem organischen Lösungsmittel,
das Vermischen der beiden Lösungen und
das Abdampfen der Lösungsmittel, um die Zusammensetzung herzustellen,
worin die Stoffwechselfehlfunktion mit anomalem Lipid- oder Glucosestoffwechsel in Zusammenhang steht.

2. Zusammensetzung zur Verwendung nach Anspruch 1, worin das Isoflavon aus der aus Genistein, Daidzein, Glycitein bestehenden Gruppe ausgewählt ist.

3. Zusammensetzung zur Verwendung nach Anspruch 1 oder Anspruch 2, worin das Isoflavon glykosyliert ist.

4. Zusammensetzung zur Verwendung nach Anspruch 3, worin das glykosylierte Isoflavon aus der aus Genistin, 6"-O-Malonylgenistin, 6"-O-Acetylgenistin, Daidzin, 6"-O-Malonyldaidzin und 6"-O-Acetyldaidzin, Glycitin, 6"-O-Malonylglycitin und 6"-O-Acetylglycitin bestehenden Gruppe ausgewählt ist.

5. Zusammensetzung zur Verwendung nach einem der vorangegangenen Ansprüche, die mehr als ein Isoflavon umfasst.

6. Zusammensetzung zur Verwendung nach einem der vorangegangenen Ansprüche, die eines oder mehrere von gesteigerter Fähigkeit, Plasmalipidspiegel zu reduzieren, gesteigerter Fähigkeit, die Expression von LDL-Rezeptoren hinaufzuregulieren, gesteigerter Fähigkeit, die Expression von Insulinrezeptoren hinaufzuregulieren, und/oder gesteigerter Insulinsensibilisierungsaktivität, in Bezug auf Isoflavon, aufweist.

7. Zusammensetzung zur Verwendung nach einem der vorangegangenen Ansprüche, die keine gesteigerte Östrogenaktivität in Bezug auf das Isoflavon aufweist.

8. Verwendung einer Zusammensetzung, die Säugetier-Molkenprotein und ein Isoflavon umfasst, bei der Herstellung eines Präparats zur Verwendung bei der Behandlung von Stoffwechselfehlfunktion im Zusammenhang mit anomalem Lipid- oder Glucosestoffwechsel, worin die Zusammensetzung durch ein Verfahren herstellbar ist, das Folgendes umfasst:
das Lösen von Säugetier-Molkenprotein in Wasser,
das Lösen eines oder mehrerer Isoflavone in einem organischen Lösungsmittel,
das Vermischen der beiden Lösungen und
das Abdampfen der Lösungsmittel, um die Zusammensetzung herzustellen.

9. Zusammensetzung zur Verwendung nach einem der Ansprüche 1 bis 7 oder Verwendung nach Anspruch 8, worin die Stoffwechselfehlfunktion aus der aus Diabetes, Adipositas, Insulinresistenz, reduzierter Glucosetoleranz, polyzystischem Ovarialsyndrom, Hypertonie, Fettleber, chronischer Hepatitis, Leberfibrose, Zirrhose, Hypercholesterinämie, Hypertriglyceridämie, Mikroalbuminurie und metabolischem Syndrom bestehenden Gruppe ausgewählt ist.

## Revendications

1. Composition comprenant une protéine de lactosérum de mammifère et une isoflavone destinée à être utilisée dans un procédé de traitement d'un dysfonctionnement métabolique associé à un métabolisme anormal des lipides ou du glucose,
où la composition peut être produite par un procédé comprenant :
la dissolution de la protéine de lactosérum de mammifère dans de l'eau,
la dissolution d'une ou de plusieurs isoflavones dans un solvant organique,
le mélange des deux solutions, et
l'évaporation des solvants afin de produire la composition,
où le dysfonctionnement métabolique est associé à un métabolisme anormal des lipides ou du glucose.

2. Composition destinée à être utilisée selon la revendication 1, dans laquelle l'isoflavone est sélectionnée dans le groupe consistant en la génistéine, la daidzéine, la glycitéine.

3. Composition destinée à être utilisée selon la revendication 1 ou la revendication 2, dans laquelle l'isoflavone est glycosylée.

4. Composition destinée à être utilisée selon la revendication 3, dans laquelle l'isoflavone glycosylée est sélectionnée dans le groupe consistant en la génistine, la 6"-O-malonylgénistine, la 6"-0-acétylgénistine, la daidzine, la 6"-O-malonyldaidzine et la 6"-O-acétyldaidzine, la glycitine, la 6"-0-malonylglycitine et la 6"-O-acétylglycitine.

5. Composition destinée à être utilisée selon l'une quelconque des revendications précédentes, qui comprend plus d'une seule isoflavone.

6. Composition destinée à être utilisée selon l'une quelconque des revendications précédentes, qui présente l'une ou plusieurs d'une aptitude accrue à réduire les taux de lipides plasmatiques, une aptitude accrue à positivement réguler l'expression du récepteur des LDL, une aptitude accrue à positivement réguler l'expression du récepteur de l'insuline et/ou une activité de sensibilisation à l'insuline accrue par rapport à une isoflavone.

7. Composition destinée à être utilisée selon l'une quelconque des revendications précédentes, qui ne présente pas d'activité oestrogénique accrue par rapport à l'isoflavone.

8. Utilisation d'une composition comprenant une protéine de lactosérum de mammifère et une isoflavone dans la fabrication d'une préparation destinée à être utilisée dans le traitement d'un dysfonctionnement métabolique associé à un métabolisme anormal des lipides ou du glucose, où la composition peut être produite par un procédé comprenant :
la dissolution de la protéine de lactosérum de mammifère dans de l'eau,
la dissolution d'une ou de plusieurs isoflavones dans un solvant organique,
le mélange des deux solutions, et
l'évaporation des solvants afin de produire la composition.

9. Composition destinée à être utilisée selon l'une quelconque des revendications 1 à 7 ou utilisation selon la revendication 8, où le dysfonctionnement métabolique est sélectionné dans le groupe consistant en le diabète, l'obésité, l'insulinorésistance, une tolérance au glucose réduite, le syndrome des ovaires polykystiques, l'hypertension, la stéatose, l'hépatite chronique, la fibrose hépatique, la cirrhose, l'hypercholestérolémie, l'hypertriglycéridémie, la microalbuminurie et un syndrome métabolique.
